(19) Europäisches Patentamt
European Patent Office
Office européen des brevets

(11) **EP 0 488 218 B1**

(12) **EUROPEAN PATENT SPECIFICATION**

(45) Date of publication and mention
of the grant of the patent:
**09.07.1997   Bulletin 1997/28**

(51) Int Cl.[6]: **C08J 3/12**

(21) Application number: **91120279.4**

(22) Date of filing: **27.11.1991**

(54) **Process for preparing polymer particles**

Verfahren zur Herstellung von Polymerteilchen

Procédé pour préparer des particules polymères

(84) Designated Contracting States:
**DE FR GB IT**

(30) Priority: **27.11.1990   US 618652**

(43) Date of publication of application:
**03.06.1992   Bulletin 1992/23**

(73) Proprietor: **United States Surgical Corporation
Norwalk, Connecticut 06856 (US)**

(72) Inventor: **Kennedy, John
Stratford, CT 06497 (US)**

(74) Representative: **Marsh, Roy David et al
Hoffmann Eitle & Partner
Patent- und Rechtsanwälte
Postfach 81 04 20
81904 München (DE)**

(56) References cited:
**EP-A- 58 481                EP-A- 237 345
EP-A- 283 925               EP-A- 0 052 793
WO-A-92/00342           CH-A- 661 206
GB-A- 1 298 121          US-A- 4 165 420
US-A- 4 384 975          US-A- 4 523 591
US-A- 4 822 534**

Remarks:
The file contains technical information submitted
after the application was filed and not included in this
specification

**Description**

BACKGROUND OF THE INVENTION

This invention relates to a process for preparing particles of polymer, namely, spheroidal particulates or beads of the bioabsorbable variety, employing an atomization technique. The particles are useful, inter alia, in the repair of damaged or defective bone.

EP-A-0 058 481 discloses a pharmaceutical composition comprising a polylactide and an acid-stable polypeptide, formulated as a solid composition for sub-dermal injection or implantation and in the form of rods, spheres, films or pellets. Polylactide includes co-polymers of lactic acid and glycolic acid, mixtures of such co-polymers, and mixtures of such co-polymers with polymers of lactic acid alone.

EP-0 237 345 discloses a method and apparatus for the rotary atomisation of meltable material at the lip of a bowl.

EP-A-0 052 793 discloses preparation of free-flowing pellets of ethylene terephthalate oligomer by water-quenching droplets of molten oligomer, to render the oligomer easily transportable in solid form.

US-A-4 165 420 discloses a process of polymerization in the solid state, in a fluidized bed of finely divided low molecular weight prepolymer particles, which are spherical beads made by atomising molten prepolymer into fine molten droplets which are then solidified.

GB-A-1 298 121 discloses an atomizing technique for making powdered thermoplastics, useful in moulding and coating applications.

The medical use of polymer particles including those of the bioabsorbable variety are known, inter alia, from U.S. Patent Nos. 3,882,858; 4,535,485; 4,547,390; 4,643,735; and 4,663,447. There has been an increase in interest in utilizing both bioabsorbable and non-absorbable particles to facilitate bone or fibrous tissue repair/reconstruction.

A number of processes are contemplated for preparing finely divided polymeric particles, e. g., mechanical grinding, solvent precipitation, dispersion, spray atomization of solutions or slurries, and rotary atomization. In rotary atomization, the polymer is applied to a rotation bell, cup or disk with mechanical forces predominating in the breakup of the polymer into particles. The Applicants have themselves previously proposed a process for preparing foamed, bioabsorbable polymer particles by a freeze-drying technique (US patent 5,102,983).

Processes which form microspheres of absorbable material less than or equal to 0.2 mm in diameter for use in controlled release of drugs, are well-known. Such spheres have generally been formed by a solvent evaporation technique. Alternatively, polymeric microspheres, e.g., beads, of average particle size greater than or equal to 0.2 mm in diameter can be formed by an emulsion polymerization process. Such emulsion polymerization has been successfully utilized to form beads of polymethylmethacrylate and styrene.

For medical applications, it is often desirable to control not only the particle size distribution of the polymeric particles but level of fibres which are present as well.

SUMMARY OF THE INVENTION

According to the present invention, there is provided a process for preparing, for medical use, particles of a bioabsorbable polymer which has a tendency to form fibers and which is derived from polyglycolic acid, glycolide, lactic acid, lactide, dioxanone, e-caprolactone, trimethylene carbonate and mixtures thereof, and has an inherent viscosity not exceeding 0.6 dl/g when measured at a temperature of 30°C, at a concentration of the polymer in chloroform or hexafluoroisopropanol, of 0.25 g/dl, while suppressing the fiber-forming tendency of the polymer, the process comprising the steps of:

a) melting the polymer;
b) a capillary extrusion step, for dividing the thus-melted polymer into particles; and
c) solidifying the particles to form microspheres having an average diameter in a range of from 0.1 to 3.0 mm.

When the conditions of the present invention are followed, particles of polymer of substantially uniform size, namely, microspheres of 0.1 to 3.0 mm average size (in diameter) can be prepared from polymeric substances, while fiber forming tendencies of the polymer are suppressed. In other words, the fiber forming tendency of the polymeric material, which is believed to relate to the surface tension exhibited by the polymer, is inhibited according to the present invention such that substantially no fibers are produced among the particles that are prepared. As used herein, the term "fiber" refers to materials which may be characterized as having a denier (see, e.g., Plastics Terms Glossary, Fourth Edition, Phillips Chemical Company, Bartlesville, Oklahoma).

The particles are preferably formed into spheres which can be used as a packing or molded part in dental or orthopedic applications in which a bony defect is filled with material. Such material then acts as a scaffold for new bony ingrowth while, in the case of bioabsorbable particles, being resorbed by the body, leaving behind a fully healed bone tissue structure.

## BRIEF DESCRIPTION OF THE DRAWINGS

The present invention will be described in greater detail below, with reference to the accompanying drawings in which:

Fig. 1 is a perspective view of an extrusion die adapter which can be utilized in accordance with the present invention; and,

Fig. 2 is a perspective view of another extrusion die adapter which can be utilized in accordance with the present invention.

## DESCRIPTION OF THE PREFERRED EMBODIMENTS

In order to minimize the formation of fibers during the process of the present invention, a polymer is selected for processing which has an inherent viscosity not exceeding 0.6 dl/g when measured at a temperature of 30°C in chloroform or HFIP (concentration of the polymer during this measurement is 0.25 g/dl). HFIP is generally used as the measuring solvent when glycolide content exceeds about 40 mole percent of the overall polymer being measured. Preferably, the polymer has an inherent viscosity, when measured under these conditions, of about 0.2 to about 0.5 dl/g, more preferably about 0.25 to about 0.45 dl/g.

It should be noted that the polymer can have an initial inherent viscosity within the levels set forth above. Alternatively, the polymer can have an initial inherent viscosity exceeding 0.6 dl/g when measured under the above conditions and then can be treated, e.g., heated, to cause degradation of the polymer, such as by hydrolysis (when heated in the presence of moisture), to reduce the viscosity to the levels set forth above. The polymer can then be further heated in accordance with the heating step of the present invention.

While not being bound by any particular theory on physical properties, it is believed that the polymer possessing an inherent viscosity not exceeding 0.6 dl/g when measured under the above conditions, exhibits a reduced tendency to form fibers because of a physical attribute associated with the polymer, e.g., an increased surface tension and/or reduced chain lengths of individual polymer chains relative to higher inherent viscosity materials. The inherent viscosity is a function of, among other factors, the molecular weight of a polymer. Accordingly, the inherent viscosity of the polymer can be controlled by selecting a polymer having an appropriate molecular weight.

The polymer is bioabsorbable and is derived from polyglycolic acid, glycolide, lactic acid, lactide, dioxanone, e-caprolactone, trimethylene carbonate and various combinations of these and related monomers. Polymers of this type are known in the art, principally as materials for the fabrication of such surgical devices as sutures, and wound clips, as disclosed in U.S. Patent Nos. 2, 668, 162; 2, 703, 316; 2,758,987; 3,225,766; 3,297,033; 3,422,181; 3,531,561; 3,565,077; 3,565,869; 3,620,218; 3,626,948; 3,636,956; 3,736,646; 3,772,420; 3,773,919; 3,792,010; 3,797,499; 3,839,297; 3,867,190; 3,878,284; 3,982,543; 4,047,533; 4,060,089; 4,137,921; 4,157,437; 4,234,775; 4,237,920; 4,300,565; and, 4,523,591; U.K. Patent No. 779,291; D.K. Gliding et al., "Biodegradable polymers for use in surgery-- polyglycolic/poly(lactic acid) homo- and co-polymers: 1", Polymer, Volume 20, pages 1459-1464 (1979), and D.F. Williams (ed.), Biocompatibility of Clinical Implant Materials, Vol. II, ch. 9: "Biodegradable Polymers" (1981).

Copolymers of glycolide and lactide with or without additional monomers are preferred and of these glycolide-lactide copolymers are most preferred.

In the process of the present invention, the polymeric material is heated so as to produce a flowable mass. The polymer is preferably heated to a temperature from 60°C to 300°C. More particularly, the temperature to which the polymeric material will be heated, will depend on the melt characteristics of the polymer selected. For example, for a glycolide/lactide copolymer, the system is heated to a temperature of from about 100 to about 300°C, preferably from about 170°C to about 270°C, and most preferably from about 220°C to about 250°C. For polymers having lower melting points, e.g., polycaprolactone, lower temperatures may be employed, e.g., about 60°C, whereas higher temperature may be required for materials having higher melting points.

After heating the polymer, the heated molten polymer is divided into particles with the molten particles then being solidified. The polymer is divided and solidified into the particles such that an average particle size (diameter) of the particles when solidified will be from 0.1 to 3.0mm, more preferably from 0.2 mm to 1.5 mm, and most preferably from 0.3 to 1.0 mm.

In this regard, the molten polymer can be divided, e.g., into droplets, by being extruded through a capillary provided in an extrusion die of extrusion apparatus. Suitable extrusion apparatus which can be utilized in accordance with the present invention are described in G.A. Kruder, "Extrusion", Encyclopedia of Polymer Science and Engineering (Second Edition), Volume 6, pages 571-631, and in P. N. Richardson, "Plastics Processing", Encyclopedia of Chemical Technology (Third Edition), Volume 18, pages 185-189. Any part of the extrusion apparatus such as the extruder screw or the capillary die can be heated to the appropriate temperature in order to heat the polymer.

As the extrusion apparatus, an Instron Rheometer available from the Instron Corp. of Canton, Massachusetts 02021 can be used. The Instron Rheometer has an extrusion barrel of about 20 cm$^3$ capacity and which is provided with a capillary die at the bottom thereof. The barrel is heated to the appropriate temperature and then loaded with the polymer, which is forced down through the capillary die by means of a plunger extending into the barrel.

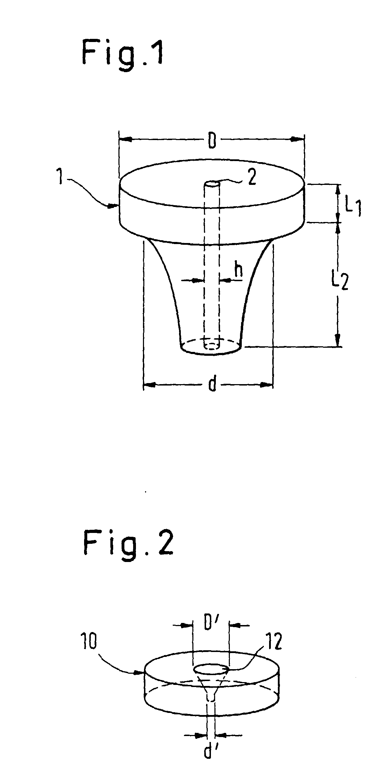

Extrusion can be carried out through a capillary die adapter 1 illustrated in Fig. 1, having a capillary 2 of substantially constant inner diameter h. Alternatively, a capillary die adapter 20 of Fig. 2 can be utilized, which comprises a capillary 12 of narrowing inner diameter. Rate of extrusion and diameter size of the capillary determine ultimate particle size of solidified polymer particles. In particular, the capillary has a narrowest inner diameter of preferably about 0.25 mm to about 0.05 mm. (about 0.010 to about 0.002 inch), more preferably about 0.23 mm to about 0.08 mm (about 0.009 to about 0.003 inch), and most preferably about 0.20 mm to about 0.10 mm (about 0.008 to about 0.004 inch). The polymer is extruded through the capillary preferably at a rate of about 380 mm/min. to about 8 mm/min. (about 15 to about 0.3 inch/min.), more preferably at a rate of about 305 mm/min to about 13 mm/min. (about 12 to about 0.5 inch/min.), and most preferably at a rate of about 254 mm/min. to about 25 mm/min. (about 10 to about 1 inch/min).

Once divided, the molten particles of polymer are solidified. Solidification can be carried out by allowing the extruded divided particles to fall into a liquid which is immiscible with the molten polymer, which freezes the polymer particles on contact therewith, and in which the solidified polymer is not soluble. The freezing liquid can be, for example, liquid nitrogen, or a mixture of solid carbon dioxide and a liquid such as acetone or pentane. In general, the temperature of the freezing liquid is advantageously at least about 10°C below the freezing temperature of the polymer particles. The lower the temperature of the freezing liquid, the faster the polymer particles will freeze solid therein. It is desirable to maintain a certain particle configuration, e.g., spheres upon freezing, and therefore rapid, even instantaneous freezing is called for. This can be conveniently achieved employing a freezing liquid such as liquid nitrogen.

The particles of frozen polymer can be recovered from the freezing liquid employing any suitable means, such as draining, straining, filtering, decanting or centrifuging.

This operation is conducted at or below the melting point of the frozen polymeric particles to maintain the particles in the frozen state.

Alternatively, the polymeric particles can be solidified by falling freely through the air. The particles are allowed to fall a distance of at least about 40 cm. through the air, whereby the particles are sufficiently cooled before striking a collecting unit so that the particles will not stick together upon striking the collecting unit. More specifically, the particles are allowed to fall a distance of preferably about 190 to about 254 cm., more preferably about 200 to about 240 cm., and most preferably about 215 to about 230 cm. before striking the collecting unit. The collecting unit may be provided, e.g., as disclosed in U.S. Patent Nos. 4,256,677 and 3,743,464.

The particles or beads formed of bioabsorbable polymers can be used as filler in a surgical prosthesis, i.e. for implantation in a cavity provided in bone or fibrous tissue to encourage regrowth and regeneration of the tissue. The particles of the bioabsorbable polymer are absorbed by the body at a predictable rate allowing tissue or bony ingrowth as absorption takes place. The rate of absorption is characteristic of the polymer utilized. Thus, e.g., a glycolide-lactide copolymer will often completely resorb within six months in contrast to about two years for polyglycolide homopolymer. Both the bioabsorbable and non-absorbable polymeric particles are readily molded to fill cavities or other contours. The beads can be heated to softening temperature, e.g., to about 60°C, at which temperature they can be worked and shaped.

Any required drug, medicinal material, or growth factor can be incorporated into the polymer prior to processing, e.g. by addition to the polymer in the customary amounts so that at the conclusion of the polymeric particle manufacturing process herein, the particles will contain a predetermined amount of one or more of such substances.

Thus, it is within the scope of this invention to incorporate one or more medico-surgically useful substances into the particles, e.g., those which accelerate or beneficially modify the healing process when particles are applied to a surgical repair site. For example, the polymer particles can carry a therapeutic agent which will be deposited at the repair site. The therapeutic agent can be chosen for its antimicrobial properties, capability for promoting repair or reconstruction and/or new tissue growth or for specific indications such as thrombosis. Antimicrobial agents such as broad spectrum antibiotics (gentamicin sulphate, erythromycin or derivatized glycopeptides) which are slowly released into the tissue can be applied in this manner to aid in combating clinical and sub-clinical infections in a tissue repair site.

To promote repair and/or tissue growth, one or several growth promoting factors can be introduced into the particles, e.g., fibroblast growth factor, bone growth factor, epidermal growth factor, platelet derived growth factor, macrophage derived growth factor, alveolar derived growth factor, monocyte derived growth factor, magainin, and so forth. Some therapeutic indications are: glycerol with tissue or kidney plasminogen activator to cause thrombosis, superoxide dismutase to scavenge tissue damaging free radicals, tumor necrosis factor for cancer therapy or colony stimulating factor and interferon, interleukin-2 or other lymphokine to enhance the immune system.

The present invention will be explained in greater detail, by way of the following examples:

EXAMPLE 1

A capillary die adapter 1 of Fig. 1 having the following dimensions: D = 18.54 mm (0.730 inch); $L_1$ = 3.175 mm (1/8 inch); $L_2$ = 3.175 mm (1/8 inch); d = 7.62 mm (0.300 inch); and h = 0.406 mm (0.016 inch), was provided on a barrel of an Instron Rheometer extrusion apparatus. The extrusion apparatus, including the barrel and the adapter 1, was heated to 250°C and then loaded with 25/75 mole percent glycolide/lactide copolymer having an inherent viscosity, measured at 30°C in HFIP and at a concentration of 0.25 g/dl, of 0.49 dl/g. This copolymer was maintained in the barrel of the extrusion apparatus for 5 minutes and then extruded through capillary 2 of the die adapter 1 at a rate of 7.62 mm/min (0.3 inch/min.), whereby droplets of the polymer were formed. These droplets of the polymer were allowed to fall from the die adapter 1 a distance of 40 cm. and into a vat of liquid nitrogen at a temperature of -196°C., with the droplets of polymer thereby solidifying into beads. The beads were collected from the liquid nitrogen, classified, and were found to have an average diameter of 3 mm. The solidified polymer thus formed was substantially free of fibers.

This procedure was repeated, but with the 25/75 mole percent glycolide/lactide copolymer being exposed to air for 72 hours before being added to the heated extruder barrel. Clearer beads of a range of particle size of 2.8-3.1 mm were produced, the solidified polymer being substantially free of fibers.

EXAMPLE 2

The procedure of Example 1 was repeated, but with a die adapter disc 10 of Fig. 2 having a capillary 12 necking down from an inner entrance diameter D' of 1.25 mm to an inner exit diameter d' of 0.203 mm (0.008 inch), with the extrusion apparatus including the adapter 10 being heated to a temperature of 225°C, and with respective rates of extrusion of 25.4 mm/min (1 inch/min.), 76.2 mm/min (3 inch/min.), and 25.4 mm/min (10 inch/min.) respectively for equal extruded amounts of the 25/75 mole percent glycolide/lactide copolymer having an inherent viscosity of 0.5 dl/g measured at 30°C in HFIP and at a concentration of 0.25 g/dl. The extrudate formed a stream that broke into droplets upon exiting from the adapter 10, with the falling droplets being directed into a bucket of liquid nitrogen at -296°C with stirring. A total of 15.65 g of beads (the product being substantially fiber free) was collected from these three combined runs and then classified, with the distribution being reported in Table II below:

Table II

| Sieve No.* | Weight (g) of Particles Retained Thereon | % of Particles Retained Thereon |
|---|---|---|
| 14 | 7.29 | 46.58 |
| 16 | 4.23 | 27.03 |
| 18 | 2.25 | 14.38 |
| 20 | 0.72 | 4.60 |
| 25 | 0.63 | 4.02 |
| 40 | 0.53 | 3.39 |
| | Total $\overline{15.65}$ g | Total $\overline{100.00}$% |

*a No. 14 sieve has openings of 1.41 mm;
a No. 16 sieve has openings of 1.19 mm;
a No. 18 sieve has openings of 1.00 mm;
a No. 20 sieve has openings of 0.841 mm;
a No. 25 sieve has openings of 0.707 mm; and
a No. 40 sieve has openings of 0.420 mm.

EXAMPLE 3

The procedure of Example 2 was repeated but with all molten polymer being extruded at a rate of 1 inch/min. A total of 6.96 g of beads was collected and then classified, with the distribution being reported in Table III below:

Table III

| Sieve No. | Weight (g) of Particles Retained Thereon | % Particles Retained Thereon |
|---|---|---|
| 14 | 0.84 | 12.07 |
| 16 | 1.71 | 24.57 |
| 18 | 1.99 | 28.59 |
| 20 | 1.83 | 26.29 |
| 25 | 0.25 | 3.59 |

Table III   (continued)

| Sieve No. | Weight (g) of Particles Retained Thereon | % Particles Retained Thereon |
|---|---|---|
| 40 | 0.30 | 4.31 |
| Passed Through 40 | 0.04 | 0.58 |
| | Total 6.96 g | Total 100.00 % |

EXAMPLE 4

The procedure of Example 2 was repeated, but with all molten polymer being extruded at a rate of 3 inches/min. A total of 13.03 g of beads was collected and then classified, with the distribution being reported in Table IV below:

Table IV

| Sieve No. | Weight (g) of Particles Retained Thereon | % Particles Retained Thereon |
|---|---|---|
| 14 | 9.93 | 76.21 |
| 16 | 2.30 | 17.65 |
| 18 | 0.64 | 4.91 |
| 20 | 0.06 | 0.46 |
| 25 | 0.07 | 0.54 |
| 40 | 0.02 | 0.15 |
| Passed Through 40 | 0.01 | 0.08 |
| | Total 13.03 g | Total 100.00 % |

EXAMPLE 5

The procedure of Example 2 was repeated, but with all molten polymer being extruded at a rate of 10 inches/min. A total of 19.62 g of beads was collected and then classified, with the distribution being reported in Table V below:

Table V

| Sieve No. | Weight (g) of Particles Retained Thereon | % Particles Retained Thereon |
|---|---|---|
| 14 | 13.49 | 68.76 |
| 16 | 3.38 | 17.23 |
| 18 | 1.35 | 6.88 |
| 20 | 0.63 | 3.21 |
| 25 | 0.38 | 1.94 |
| 40 | 0.32 | 1.63 |
| Passed Through 40 | 0.07 | 0.36 |
| | Total 19.62 g | Total 100.01 %** |

**100.01% value due to rounding of significant figures.

EXAMPLE 6

The procedure of Example 3 was repeated in-its entirety, but with the molten polymer being extruded through die adapter 10 having an extrusion channel 12 necking down from an entrance diameter D' of 1.25 mm to a minimum a diameter d' of 0.152 mm (0.006 inch) at the outlet thereof. A total of 5.94 g of beads was collected and then classified, with the distribution being reported in Table VI below:

Table VI

| Sieve No. | Weight (g) of Particles Retained Thereon | % Particles Retained Thereon |
|---|---|---|
| 14 | 0.70 | 11.78 |
| 16 | 1.09 | 18.35 |
| 18 | 1.45 | 24.41 |
| 20 | 1.17 | 19.70 |

Table VI   (continued)

| Sieve No. | Weight (g) of Particles Retained Thereon | % Particles Retained Thereon |
|---|---|---|
| 25 | 0.88 | 14.82 |
| 40 | 0.58 | 9.76 |
| Passed Through 40 | 0.07 | 1.18 |
| | Total 5.94 g | Total 100.00 % |

EXAMPLE 7

The procedure of Example 4 was repeated in its entirety, but with the molten polymer being extruded through die adapter 10 having an extrusion channel 12 necking down from an entrance diameter D' of 1.25 mm to a minimum diameter d' of 0.152 mm (0.006 inch) at the outlet thereof. A total of 8.49 g of beads was collected and then classified, with the distribution being reported in Table VII below:

Table VII

| Sieve No. | Weight (g) of Particles Retained Thereon | % Particles Retained Thereon |
|---|---|---|
| 14 | 2.58 | 30.39 |
| 16 | 2.89 | 34.04 |
| 18 | 2.15 | 25.32 |
| 20 | 0.39 | 4.59 |
| 25 | 0.23 | 2.71 |
| 40 | 0.18 | 2.12 |
| Passed Through 40 | 0.07 | 0.83 |
| | Total 8.49 g | Total 100.00 % |

EXAMPLE 8

The procedure of Example 5 was repeated in its entirety, but with the molten polymer being extruded through a die adapter 10 having an extrusion channel 12 necking down from an entrance diameter D' of 1.25 mm to a minimum diameter d' of 0.152 mm (0.006 inch) at the outlet thereof. A total of 14.5 g of beads was collected and then classified, with the distribution being reported in Table VIII below:

Table VIII

| Sieve No. | Weight (g) of Particles Retained Thereon | % Particles Retained Thereon |
|---|---|---|
| 14 | 5.63 | 38.83 |
| 16 | 5.06 | 34.90 |
| 18 | 1.52 | 10.48 |
| 20 | 0.72 | 4.96 |
| 25 | 0.82 | 5.66 |
| 40 | 0.63 | 4.34 |
| Passed Through 40 | 0.12 | 0.83 |
| | Total 14.50 g | Total 100.00 % |

EXAMPLE 9

The procedure of Example 2 was repeated in its entirety, but with the molten polymer additionally being stirred before extrusion. A total of 56.77 g of beads was collected and then classified, with the distribution being reported in Table IX below:

Table IX

| Sieve No. | Weight (g) of Particles Retained Thereon | % of Particles Retained Thereon |
|---|---|---|
| 14 | 22.36 | 39.39 |

Table IX   (continued)

| Sieve No. | Weight (g) of Particles Retained Thereon | % of Particles Retained Thereon |
|---|---|---|
| 16 | 17.00 | 29.95 |
| 18 | 11.78 | 20.75 |
| 20 | 1.99 | 3.51 |
| 25 | 1.66 | 2.92 |
| 40 | 1.62 | 2.85 |
| Passed Through 40 | 0.36 | 0.63 |
| | Total 56.77 g | Total 100.00 % |

## EXAMPLE 10

The procedure of Example 2 was repeated in its entirety, but with a die 10 having a channel 12 necking down from an entrance diameter D' of 1.25 mm to a minimum exit diameter d' of 0.152 mm (0.006 inch). A total of 7.61g of beads was collected and then classified, with the distribution being reported in Table X below:

Table X

| Sieve No. | Weight (g) of Particles Retained Thereon | % Particles Retained Thereon |
|---|---|---|
| 20 | 0.32 | 4.20 |
| 25 | 3.72 | 48.88 |
| 40 | 3.39 | 44.55 |
| Passed Through 40 | 0.18 | 2.37 |
| | Total 7.61 g | Total 100.00 % |

## EXAMPLE 11

The procedure of Example 7 was repeated in its entirety, but with polyglycolic acid (PGA), having an inherent viscosity of 0.25 dl/g measured at 30°C in HFIP and at a concentration of 0.25 g/dl, being heated to 225°C and then being extruded at a rate of 76.2 mm/min (3 inch/min). A total of 10.04 g of PGA beads was collected and then classified, with the distribution being reported in Table XI below:

Table XI

| Sieve No. | Weight (g) of Particles Retained Thereon | % Particles Retained Thereon |
|---|---|---|
| 20 | 6.30 | 62.75 |
| 25 | 1.80 | 17.93 |
| 40 | 1.70 | 16.93 |
| Passed Through 40 | 0.24 | 2.39 |
| | Total 10.04 g | Total 100.00 % |

## EXAMPLE 12

The procedure of Example 11 was repeated, but with the polyglycolic acid (PGA) being heated to 240°C and then being extruded (the PGA had an inherent viscosity of 0.25 dl/g at 30°C in HFIP and at a concentration of 0.25 g/dl). A total of 5.52 g of PGA beads was collected and then classified, with the results being reported in Table XII below:

Table XII

| Sieve No. | Weight (g) of Particles Retained Thereon | % Particles Retained Thereon |
|---|---|---|
| 20 | 4.90 | 88.77 |
| 25 | 0.22 | 3.98 |
| 40 | 0.31 | 5.62 |
| Passed Through 40 | 0.09 | 1.63 |
| | Total 5.52 g | Total 100.00 % |

## Claims

1. A process for preparing, for medical use, particles of a bioabsorbable polymer which has a tendency to form fibers and which is derived from polyglycolic acid, glycolide, lactic acid, lactide, dioxanone, e-caprolactone, trimethylene carbonate and mixtures thereof, and has an inherent viscosity not exceeding 0.6 dl/g when measured at a temperature of 30°C, at a concentration of the polymer in chloroform or hexafluoroisopropanol, of 0.25 g/dl, while suppressing the fiber-forming tendency of the polymer, the process comprising the steps of:

   a) melting the polymer;
   b) a capillary extrusion step, for dividing the thus-melted polymer into particles; and
   c) solidifying the particles to form microspheres having an average diameter in a range of from 0.1 to 3.0 mm.

2. A process according to claim 1, wherein the polymer has an initial inherent viscosity above 0.6 dl/g when measured at a temperature of 30°C in chloroform or hexafluoroisopropanol, and further comprising:
   treating the polymer so that the viscosity of the polymer is reduced to a level not exceeding 0.6 dl/g when measured at 30°C in chloroform or hexafluoroisopropanol and then heating the polymer in accordance with step (a).

3. A process according to claim 1 or 2 wherein the polymer is melted by heating to a temperature in a range of from 60°C to 300°C.

4. A process according to any one of the preceding claims, wherein the particles are solidified by being introduced into liquid which is immiscible with the polymer and which freezes the particles on contact therewith.

5. A process according to any one of claims 1 to 4, wherein the polymer particles are solidified by falling freely through air.

6. A process according to any one of claims 1 to 5, wherein the bioabsorbable polymer is a co-polymer of glycolide and lactide with or without additional monomer.

## Patentansprüche

1. Verfahren zum Herstellen zur medizinischen Verwendung von Partikeln eines bioabsorbierbaren Polymers, das eine Neigung besitzt, Fasern zu bilden, und das aus Polyglycolsäure, Glycolid, Milchsäure, Lactid, Dioxanon, e-Caprolacton, Trimethylencarbonat und Mischungen derselben stammt und eine innere Viskosität besitzt, die nicht 0,6 dl/g überschreitet, wenn diese bei einer Temperatur von 30°C bei einer Konzentration des Polymers in Chloroform oder Hexafluorisopropanol von 0,25 g/dl gemessen ist, während die Neigung zum Bilden von Fasern des Polymers unterdrückt wird, wobei das Verfahren die Schritte umfaßt:

   a) Schmelzen des Polymers;
   b) einen Schritt der Kapillarextrusion, um das so geschmolzene Polymer in Partikel zu unterteilen; und
   c) in Feststofform Überführen der Partikel, um Mikrokugeln mit einem durchschnittlichen Durchmesser in einem Bereich von 0,1 bis 3,0 mm zu bilden.

2. Verfahren gemäß Anspruch 1, wobei das Polymer eine anfängliche innere Viskosität über 0,6 dl/g besitzt, wenn sie bei einer Temperatur von 30°C in Chloroform oder Hexafluorisopropanol gemessen wird und weiter umfassend:

   - Behandeln des Polymers, so daß die Viskosität des Polymers auf ein Maß verringert wird, das nicht 0,6 dl/g überschreitet, wenn sie bei 30°C in Chloroform oder Hexafluorisopropanol gemessen wird, und dann Erhitzen des Polymers gemäß Schritt (a).

3. Verfahren gemäß Anspruch 1 oder 2, wobei das Polymer durch Erhitzen auf eine Temperatur in einem Bereich von 60°C bis 300°C geschmolzen wird.

4. Verfahren gemäß einem der vorhergehenden Ansprüche, wobei die Partikel in Feststofform überführt werden, indem sie in eine Flüssigkeit eingeführt werden, die mit dem Polymer nicht mischbar ist und welche die Partikel beim Kontakt mit dieser einfriert.

**5.** Verfahren gemäß einem der Ansprüche 1 bis 4, wobei die Polymerpartikel in Feststofform überführt werden, indem sie frei durch Luft fallen.

**6.** Verfahren gemäß einem der Ansprüche 1 bis 5, wobei das bioabsorbierbare Polymer ein Copolymer aus Glycolid und Lactid mit oder ohne zusätzliches Monomer ist.

**Revendications**

**1.** Procédé de préparation, pour un usage médical, de particules d'un polymère bioabsorbable qui a tendance à former des fibres et qui est dérivé de l'acide polyglycolique, du glycolide, de l'acide lactique, du lactide, de la dioxanone, de la e-caprolactone, du triméthylène carbonate et de leurs mélanges, et qui a une viscosité inhérente qui ne dépasse pas 0,6 dl/g en mesurant à une température de 30°C à une concentration du polymère dans le chloroforme ou l'hexafluoroisopropanol de 0,25 g/dl, tout en supprimant la tendance du polymère à former des fibres, le procédé comprenant les étapes de :

a) faire fondre le polymère ;
b) une étape d'extrusion capillaire, pour diviser le polymère ainsi fondu en particules ;
c) solidifier les particules pour former des microsphères ayant un diamètre moyen compris entre 0,1 et 3,0 mm.

**2.** Procédé selon la revendication 1, où le polymère a une viscosité inhérente initiale supérieure à 0,6 dl/g en mesurant à une température de 30°C dans le chloroforme ou l'hexafluoroisopropanol et comprenant de plus :
le traitement de polymère de façon que la viscosité du polymère soit réduite à un niveau ne dépassant pas 0,6 dl/g en mesurant à 30°C dans le chloroforme ou l'hexafluoroisopropanol puis en chauffant le polymère selon l'étape (a).

**3.** Procédé selon la revendication 1 ou 2, où le polymère est fondu en le chauffant à une température comprise entre 60°C et 300°C.

**4.** Procédé selon l'une quelconque des revendications précédentes, où les particules sont solidifiées en étant introduites dans un liquide qui est immiscible avec le polymère et qui congèle les particules lors d'un contact avec elles.

**5.** Procédé selon l'une quelconque des revendications 1 à 4 où les particules du polymère sont solidifiées en tombant librement à travers l'air.

**6.** Procédé selon l'une quelconque des revendications 1 à 5, où le polymère bioabsorbable est un copolymère de glycolide et de lactide avec ou sans monomère additionnel.

# Fig.1

# Fig.2